(19) Europäisches Patentamt
European Patent Office
Office européen des brevets

(11) Veröffentlichungsnummer: **0 197 404**
**A2**

(12) # EUROPÄISCHE PATENTANMELDUNG

(21) Anmeldenummer: 86103943.6

(22) Anmeldetag: 22.03.86

(51) Int. Cl.[4]: C 07 C 45/64
C 07 C 49/82, B 01 F 17/42

(30) Priorität: 06.04.85 DE 3512541

(43) Veröffentlichungstag der Anmeldung:
15.10.86 Patentblatt 86/42

(84) Benannte Vertragsstaaten:
AT BE CH DE FR GB IT LI NL SE

(71) Anmelder: Merck Patent Gesellschaft mit beschränkter Haftung
Frankfurter Strasse 250
D-6100 Darmstadt(DE)

(72) Erfinder: Köhler, Manfred, Dr.
Hebbelstrasse 38
D-6100 Darmstadt(DE)

(72) Erfinder: Römer, Michael, Dr.
Im grossen Garten 18
D-6054 Rodgau 2(DE)

(72) Erfinder: Herz, Claus P., Dr.
Berghalde 20
D-6900 Heidelberg(DE)

(54) **Verfahren zur Herstellung von Ketonen.**

(57) Ketone der Formel II,

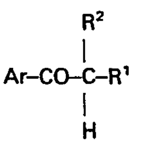

II

mit der im Patentanspruch 1 für Ar, $R^1$ und $R^2$ angegebenen Bedeutung, lassen sich mit Tetrahalogenmethanen und Alkalimetallhydroxiden unter Zusatz eines Phasentransferreagenzes leicht und in hohen Ausbeuten in α-Stellung hydroxylieren.

EP 0 197 404 A2

Merck Patent Gesellschaft
mit beschränkter Haftung

6100 D a r m s t a d t

# Verfahren zur Herstellung von Ketonen

Die Erfindung betrifft ein Verfahren zur Herstellung α-hydroxylierter Ketone aus Ketonen mit einem Tetrahalogenmethan und Alkalimetallhydroxid unter Phasentransferbedingungen.

α-Hydroxylierte Ketone eignen sich als Initiatoren für die Photopolymerisation ungesättigter Verbindungen wie z. B. in der DE-PS 27 22 264 beschrieben.

Es ist eine Reihe von Verfahren zur Herstellung α-hydroxylierter Ketone bekannt. Eine Übersicht findet sich in Houben-Weyl, Methoden der Organischen Chemie, Georg-Thieme-Verlag, Stuttgart, Bd. VII/2c und in der oben aufgeführten Patentschrift.

Die bisher bekannten Verfahren weisen eine Reihe von Nachteilen auf. So werden Ketone der Formel II mit z. B. Chlor oder Brom in die entsprechenden α-Halogenketone überführt. Diese lassen sich mit Metallalkoholaten zu α-Alkoxyepoxiden umsetzen, deren Hydrolyse die gewünschten α-hydroxylierten Ketone der Formel I liefert. Dabei entstehende vielfältige Nebenprodukte verringern jedoch die Ausbeute an gewünschtem Produkt.

α-Halogenketone können unter basischen Bedingungen direkt zu α-hydroxylierten Ketonen hydrolysiert werden, wobei diese vielfach basenkatalysierte Isomerisierungen oder Faworsky-Umlagerungen erleiden. Solcherart entstandene Nebenprodukte führen ebenfalls zu Ausbeuteminderungen und Qualitätseinbußen beim Verfahrensprodukt.

Aus der EP-OS 0 094 347 ist ein Verfahren zur Herstellung von α-hydroxylierten Ketonen bekannt, bei dem α-Halogenketone mit Hydroxidionen erzeugenden Verbindungen in Gegenwart eines Phasentransferkatalysators umgesetzt werden. Die erforderlichen Halogenketone müssen zuvor in einem zusätzlichen Verfahrensschritt aus den Ketonen der Formel II bereitet werden. Neben vermehrtem Arbeits- und Kostenaufwand ist die Herstellung der α-Halogenketone häufig aufgrund von die Reaktionsbedingungen nicht tolerierenden Substituenten der Ausgangsverbindungen mit Nebenproduktbildung und daraus resultierender Minderausbeute verbunden.

V. M. Kolb, Dissertation Southern Illinois University, Carbondale, 1976, beschreibt ein Verfahren zur direkten α-Hydroxylierung von Ketonen durch Umsetzung mit Tetrachlorkohlenstoff und gepulvertem Kaliumhydroxid in tert. Butanol als Lösungsmittel. Neben meist nur mäßigen Ausbeuten erleiden Hydroxyketone der Formel I unter den beschriebenen Reaktionsbedingungen leicht Umlagerungen. So wird anstelle von 1-Phenyl-2-hydroxy-2-methylpropan-1-on über die Zwischenstufe des cis-2,2,5,5-Tetramethyl-3,6-dihydroxy-3,6-diphenyl-1,4-dioxans unter Wasserabspaltung gebildetes 3,3,6,6-Tetramethyl-1,4-diphenyl-2,5,7-trioxabicyclo[2.2.1]heptan isoliert. Nachteilig ist auch die Erfordernis, auf die Verwendung gepulverten und damit teuren und schlecht zu handhabenden Kaliumhydroxids als Hydroxidionen erzeugender Verbindung beschränkt zu sein.

Es besteht somit ein Bedürfnis nach einem Verfahren, welches die Herstellung α-hydroxylierter Ketone der Formel I aus Ketonen der Formel II ohne zusätzliche Zwischenstufen in hohen Ausbeuten ohne Nebenproduktbildung in stabiler Form und unter Verwendung wohlfeiler Hilfsstoffe erlaubt.

Diese Aufgabe wird gelöst durch das erfindungsgemäße Verfahren zur Herstellung von Ketonen.

Gegenstand der Erfindung ist daher ein Verfahren zur Herstellung von Ketonen der allgemeinen Formel I,

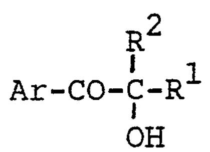

I

worin

Ar Aryl mit 6-14 C-Atomen oder durch $C_1$-$C_{12}$ Alkyl, $C_1$-$C_4$ Alkoxy, $C_1$-$C_4$ Alkylthio, Cl, Br oder CN substituiertes Aryl,

$R^1$ und $R^2$ gleich oder verschieden sind und jeweils $C_1$-$C_7$ Alkyl, $C_3$-$C_8$ Cycloalkyl, oder zusammen mit dem C-Atom, an das sie gebunden sind, $C_3$-$C_9$ Cycloalkyl

bedeuten, dadurch gekennzeichnet, daß man ein Keton der allgemeinen Formel II,

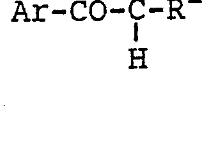

II

mit der für Ar, $R^1$ und $R^2$ oben angegebenen Bedeutung mit einem Tetrahalogenmethan der allgemeinen Formel III,

$$CF_aCl_bBr_c \qquad III$$

worin b und c 0-4, a 0-2 bedeuten und a + b + c = 4, und einem Alkalimetallhydroxid unter Phasentransferbedingungen umsetzt.

Die Durchführung der Reaktion gestaltet sich einfach. Die als Ausgangsmaterial einzusetzenden Ketone der Formel II sind bekannt oder können nach an sich bekannten Methoden wie sie in der Literatur (z. B. in Standardwerken wie Houben-Weyl, Methoden der Organischen Chemie, Georg-Thieme-Verlag, Stuttgart, Bd. VII) beschrieben sind, und zwar unter Reaktionsbedingungen, wie sie für die genannten Umsetzungen bekannt und geeignet sind, hergestellt werden. Dabei kann auch von an sich bekannten, hier nicht näher erwähnten Varianten Gebrauch gemacht werden. In den zu hydroxylierenden Ketonen der Formel II bedeutet Ar vorzugsweise Phenyl oder substituiertes Phenyl, insbesondere Phenyl, 4-Methylphenyl, 4-Ethylphenyl, 4-Isopropylphenyl, 4-Dodecylphenyl, 3,4-Dimethylphenyl, 4-Methoxyphenyl oder 4-Chlorphenyl. $R^1$ und $R^2$ bedeuten vorzugsweise $C_1$-$C_4$ Alkyl, $C_3$-$C_6$ Cycloalkyl oder zusammen mit dem C-Atom, an das sie gebunden sind, $C_3$-$C_6$ Cycloalkyl, insbesondere Methyl, Ethyl, Propyl, Butyl, Cyclohexyl oder zusammen mit dem C-Atom, an das sie gebunden sind, Cyclohexyl.

In den Tetrahalogenmethanen der Formel III ist a vorzugsweise 0, insbesondere a = c = 0, b = 4 oder a = b = 0, c = 4; ganz besonders bevorzugt ist Tetrachlorkohlenstoff.

Geeignete Alkalimetallhydroxide sind die Hydroxide des Lithiums, Natriums, Kaliums, Rubidiums und Cesiums, vorzugsweise die des Natriums und Kaliums, insbesondere Natriumhydroxid. Zwei-Phasen-Reaktionen mit einem Phasenübergang fest-flüssig erfordern zur Erzielung annehmbarer Umsatzraten üblicherweise eine möglichst große Oberfläche der festen Phase. Beispielsweise gelingt nach der U.S.-Patentschrift 3 830 862 die Eliminierung von Dibenzylsulfon im System Kaliumhydroxid/Tetrachlorkohlenstoff nur mit fein gepulvertem Kaliumhydroxid und nicht mit handelsüblichen Plätzchen. Es war daher umso überraschender, daß auch mit den wesentlich leichter zu handhabenden Plätzchen-, Granulat- oder Schuppenformen der Alkalimetallhydroxide sehr gute Ergebnisse erzielt werden. Weiterhin können die Alkalimetallhydroxide auch gepulvert oder auf feste Träger aufgebracht sein.

Die als Phasentransferreagenzien eingesetzten Verbindungen werden aus den Gruppen der quartären Ammoniumverbindungen, der Oligo- oder Polyethylenglykole oder deren Mono- oder Bisether oder der cyclischen Polyethylenglykolether ("Kronenether") ausgewählt.

In den quartären Ammoniumverbindungen der Formel IV sind die Reste $R^3$, $R^4$, $R^5$ und $R^6$ gleich oder verschieden und bedeuten geradkettiges oder verzweigtes Alkyl mit zusammen 4 bis 30 C-Atomen oder Benzyl. X bedeutet ein geeignetes Anion wie beispielsweise F, Cl, CN, OH, $HSO_4$ oder $CH_3OSO_3$. Die Wahl des Anions ist nicht kritisch; es können alle ein- oder mehrfach geladenen Anionen

eingesetzt werden. Besonders bevorzugt sind Benzyltrimethylammoniumhydrogensulfat und Methyltrioctylammoniumchlorid. Gegebenenfalls können die quartären Ammoniumverbindungen der Formel IV auch durch chemische Bindung an einen polymeren flüssigen oder festen Träger immobilisiert sein.

Eine bevorzugte Gruppe von Phasentransferreagenzien stellen die Oligo- und Polyethylenglykole und deren Mono- oder Bisether dar, die der allgemeinen Formel $R^7O-(CH_2-CH_2O-)_nR^8$ entsprechen, in der n gleich oder größer als 2 ist und $R^7$ und $R^8$ gleich oder verschieden sind und H, Alkyl-, Aryl- und/oder Cycloalkylgruppen bedeuten.

$R^7$ und/oder $R^8$ können H und/oder verzweigte und unverzweigte Alkylreste mit 1 bis 20, vorzugsweise 1 bis 10 C-Atomen, Cycloalkylreste mit 3 bis 15, vorzugsweise mit 5 bis 10 C-Atomen, und/oder Arylreste mit 6 bis 14, vorzugsweise mit 6 bis 10 C-Atomen, insbesondere Phenyl und substituiertes Phenyl sein.

Es können sowohl einheitliche Verbindungen wie z. B. Hexaethylenglykolmono- und -dimethylether, Octaethylenglykolmono- und -diethylether und Decaethylenglykolmono- und -didecylether als auch Mischungen von Ethern mit verschiedenem Oligomerisationsgrad und entsprechenden mittleren Molekulargewichten als Lösungsmittel verwendet werden, wie z. B. Polyethylenglykolmono- und -dimethylether (n = 7,9; mittleres Molekulargewicht 400) und Polyethylenglykolmono- und -dimethylether (n = 22,3; mittleres Molekulargewicht 1000).

Besonders vorteilhaft sind aromatische Oligoethylenglykolmonoether, insbesondere Decaethylenglykolmonoarylether und substituierte Decaethylenglykolmonoarylether.

Eine besonders geeignete Verbindung ist Decaethylenglykolmono-(4-nonyl-phenyl)-ether, die damit ein bevorzugtes Phasentransferreagenz im erfindungsgemäßen Verfahren zur Herstellung von Ketonen darstellt.

Das molare Verhältnis von Tetrahalogenmethan und Alkalimetallhydroxid bezogen auf das als Substrat eingesetzte Keton der Formel II beträgt mindestens jeweils 1 : 1, vorzugsweise 1 bis 2 : 1.

Die Menge an Phasentransferreagenz kann in weiten Grenzen beliebig gewählt werden, vorzugsweise liegt sie bei 10 bis 100 Gew. % bezogen auf eingesetztes Keton.

Die Ausführung der Reaktion erfolgt durch Vermischen der Komponenten Keton der Formel II, Tetrahalogenmethan der Formel III und Alkalimetallhydroxid sowie Erwärmen des gerührten Reaktionsgemisches auf die zur Umsetzung erforderliche Temperatur, wobei es jedoch von Vorteil sein kann, das Alkalimetallhydroxid langsam zu den bereits vorgelegten anderen Komponenten zuzugeben. Üblicherweise liegen die Reaktionstemperaturen bei 0 bis 100°, vorzugsweise bei 10 bis 50°. Die Reaktionszeiten betragen 1 bis 60 Stunden, vorzugsweise 12 bis 48 Stunden.

Die Anwendung von Ultraschall, wie z.B. von Lucke und Damiano, J.Amer.Chem.Soc. 102 (1980) 7926 für die Grignardreaktion beschrieben, kann den Reaktionsverlauf positiv beeinflussen.

Zur Verringerung der Viskosität des Reaktionsgemisches, zur Verminderung der Reaktivität besonders umsatzfreudiger Komponenten oder auch zur Reduktion der Menge an Phasentransferreagenz kann dem Reaktionsgemisch ein weiteres inertes organisches Lösungsmittel zugesetzt werden.

Zweckmäßigerweise trifft man die Auswahl aus den inerten, unter den gewählten Reaktionsbedingungen stabilen Lösungsmitteln, wie z.B. aliphatische oder aromatische Kohlenwasserstoffe, insbesondere Cyclohexan, Benzol oder Toluol, und aliphatische Ether, insbesondere Tetrahydrofuran oder Dioxan. Es können auch Gemische dieser Lösungsmittel zugesetzt werden.

Nach Durchführung der Reaktion fügt man dem Reaktionsgemisch zur Aufarbeitung zweckmäßig Wasser zu und isoliert das gewünschte Produkt durch Extraktion mit anschließender Destillation, Chromatographie oder Kristallisation. Auf diese Weise können die eingesetzten Ketone einfach und in hohen Ausbeuten α-hydroxyliert werden. Der hauptsächlich aus unverändertem Phasentransferreagenz bestehende Aufarbeitungsrückstand kann bei Entfaltung unveränderter Reaktivität für einen erneuten Reaktionsansatz wiederverwendet werden.

In einer besonders bevorzugten Ausführungsform der Erfindung werden aus 1-Phenyl-2-methylpropan-1-on, 1-(4-Isopropylphenyl)-2-methylpropan-1-on oder Cyclohexylphenylketon mit Tetrachlorkohlenstoff, Natriumhydroxid und Decaethylenglykolmono-(4-nonyl-phenyl)-ether, gegebenenfalls unter Zusatz eines inerten Lösungsmittels, als Initiator für die Photopolymerisation ungesättigter Verbindungen verwendbares 1-Phenyl-2-hydroxy-2-methyl-propan-1-on, 1-(4-Isopropylphenyl)-2-hydroxy-2-methyl-propan-1-on und (1-Hydroxycyclohexyl)-phenylketon hergestellt.

In der vorliegenden Erfindung steht somit ein sehr vorteilhaftes Verfahren zur einfachen und in hohen Ausbeuten verlaufenden Herstellung von α-hydroxylierten Ketonen zur Verfügung.

Beispiel 1

Zu dem Gemisch von 895 g 1-Phenyl-2-methylpropan-1-on, 900 ml Tetrachlorkohlenstoff, 1,8 Toluol und 300 ml Decaethylenglykolmono-(4-nonylphenyl)-ether werden bei 15° unter Rühren innerhalb von 48 Stunden insgesamt 552 g Natriumhydroxid portionsweise zugegeben.

Zur Aufarbeitung wird mit 3 Toluol versetzt und mit 1,8 Wasser ausgerührt. Man trennt die organische Phase ab und befreit unter vermindertem Druck vom Lösungsmittel. Der Rückstand wird der fraktionierenden Destillation unterworfen. Nach einem geringen Vorlauf gehen bei 50 bis 100 Pa und 75 - 85° 877 g (89 % d. Th.) praktisch farbloses 1-Phenyl-2-hydroxy-2-methylpropan-1-on mit einem gaschromatographisch ermittelten Gehalt von 98,5 % über.

Der das Phasentransferreagenz enthaltende Destillationsrückstand kann in einem weiteren Reaktionsansatz mit unveränderter Aktivität erneut verwendet werden.

Beispiel 2

Zu dem Gemisch aus 74,5 g 1-Phenyl-2-methylpropan-1-on, 75 ml Tetrachlorkohlenstoff, 150 ml Toluol und 1 g Dibenzo-18-krone-6 (Octahydrodibenzo[b,k]-1,4,7,10,13,16-hexaoxacyclooctadecen) werden bei 15° unter Rühren innerhalb von 40 Stunden insgesamt 44 g Natriumhydroxid in Plätzchenform zugegeben.

Man arbeitet analog zu Beispiel 1 auf und erhält nach Destillation 65,3 g (78 % d. Th.) reines 1-Phenyl-2-hydroxy-2-methylpropan-1-on.

Beispiel 3

In das Gemisch aus 74,5 g 1-Phenyl-2-methylpropan-1-on, 75 ml Tetrachlorkohlenstoff, 150 ml Toluol und 25 ml Methyltrioctylammoniumchlorid werden bei 15° unter Rühren innerhalb von 20 Stunden insgesamt 44 g granuliertes Natriumhydroxid eingetragen.

Nach Aufarbeitung wie in Beispiel 1 beschrieben und destillativer Reinigung werden 62,1 g (74 % d. Th.) reines 1-Phenyl-2-hydroxy-2-methylpropan-1-on erhalten.

Merck Patent Gesellschaft
mit beschränkter Haftung

6100 Darmstadt

Patentansprüche

1. Verfahren zur Herstellung von Ketonen der allgemeinen Formel I,

$$Ar-CO-\overset{\displaystyle R^2}{\underset{\displaystyle OH}{\overset{|}{\underset{|}{C}}}}-R^1 \qquad I$$

worin

Ar Aryl mit 6-14 C-Atomen oder durch $C_1$-$C_{12}$ Alkyl, $C_1$-$C_4$ Alkoxy, $C_1$-$C_4$ Alkylthio, Cl, Br oder CN substituiertes Aryl,

$R^1$ und $R^2$ gleich oder verschieden sind und jeweils $C_1$-$C_7$ Alkyl, $C_3$-$C_8$ Cycloalkyl, oder zusammen mit dem C-Atom, an das sie gebunden sind, $C_3$-$C_9$ Cycloalkyl

bedeuten, dadurch gekennzeichnet, daß man ein Keton der allgemeinen Formel II,

$$Ar-CO-\overset{\displaystyle R^2}{\underset{\displaystyle H}{\overset{|}{\underset{|}{C}}}}-R^1 \qquad II$$

mit der für Ar, $R^1$ und $R^2$ oben angegebenen Bedeutung mit einem Tetrahalogenmethan der allgemeinen Formel III,

$$CF_aCl_bBr_c \qquad III$$

worin b und c 0-4, a 0-2 bedeuten und a + b + c = 4, und einem Alkalimetallhydroxid unter Phasentransferbedingungen umsetzt.

2. Verfahren nach Anspruch 1, dadurch gekennzeichnet, daß in Formel III a = c = 0 und b = 4 gilt.

3. Verfahren nach einem der Ansprüche 1 und 2, dadurch gekennzeichnet, daß man als Alkalimetallhydroxid Natriumhydroxid verwendet.

4. Verfahren nach einem der Ansprüche 1 bis 3, dadurch gekennzeichnet, daß man als Phasentransferreagenz Oligo- oder Polyethylenglykole oder deren Mono- oder Bisether, cyclische Polyethylenglykolether und/oder quartäre Ammoniumverbindungen der allgemeinen Formel IV,

$$R^3R^4R^5R^6N^{\oplus}X^{\ominus} \qquad IV$$

worin $R^3$, $R^4$, $R^5$, $R^6$ gleich oder verschieden sind und geradkettiges oder verzweigtes Alkyl, wobei alle 4 Alkylgruppen zusammen 4 bis 30 C-Atome besitzen, oder Benzyl bedeuten, und X ein ein- oder mehrfach geladenes Anion bedeuted, verwendet.

5. Verfahren nach Anspruch 4, dadurch gekennzeichnet, daß man als Phasentransferreagenz einen Decaethylenglykolmonoarylether verwendet.

6. Verfahren nach Anspruch 4, dadurch gekennzeichnet, daß man als Phasentransferreagenz Benzyltrimethylammoniumhydroxid, Tetrabutylammoniumchlorid, Butyltriethylammoniumhydrogensulfat oder Methyltrioctylammoniumchlorid verwendet.

7. Verfahren nach einem der Ansprüche 4 und 6, dadurch gekennzeichnet, daß die als Phasentransferreagenz eingesetzte quartäre Ammoniumverbindung der allgemeinen Formel IV polymer gebunden ist.

8. Verfahren nach einem der Ansprüche 1 bis 7, dadurch gekennzeichnet, daß man 1-Phenyl-2-hydroxy-2-methylpropan-1-on, 1-(4-Isopropylphenyl)-2-hydroxy-2-methylpropan-1-on und (1-Hydroxycyclohexyl)-phenylketon herstellt, indem man als entsprechendes Keton der Formel II 1-Phenyl-2-methylpropan-1-on, 1-(4-Isopropylphenyl)-2-methylpropan-1-on oder Cyclohexylphenylketon einsetzt.